# EUROPEAN PATENT APPLICATION

(11) **EP 1 800 701 A1**
(43) Date of publication of application: **27.06.2007**
(21) Application number: 06256452.1
(22) Date of filing: 19.12.2006
(51) Int. Cl.: A61L 27/44, A61F 2/30

(54) **Fiber-reinforced water-swellable articles**

(30) Priority: 20.12.2005 US 751852 P
(71) Applicant: ZIMMER INC., Warsaw, Indiana 46580-2746 (US)
(72) Inventor: Zhang, Kai, Warsaw, Indiana 46582 (US); Thomas, Brian, Colombia City, Indiana 46725 (US)
(74) Representative: Findlay, Alice Rosemary

(57) **Abstract**

This invention provides water-swellable materials including polymeric fibers dispersed in a water-swellable polymer, and fiber-reinforced articles made from such water-swellable materials. The invention also provides a method of forming a fiber-reinforced, water-swellable material. These water-swellable materials and fiber-reinforced articles may be used in biomedical and pharmaceutical applications and may be suitable for implanted joint repair materials such as an articulating or bearing surface in an implanted hip, knee, spine, finger, elbow or shoulder joint.

## Description

The present invention relates to water-swellable materials that include polymeric fibers that may be suitable for use in biomedical or other applications.

Hydrogels are water-swellable or water-swollen materials having a structure defined by a crosslinked network of hydrophilic homopolymers or copolymers. The hydrophilic homopolymers or copolymers may or may not be water-soluble in free form, but in a hydrogel are rendered insoluble (but swellable) in water due to covalent, ionic, or physical crosslinking. In the case of physical crosslinking, the linking may take the form of entanglements, crystallites, or hydrogen-bonded structures. The crosslinks in a hydrogel provide structure and physical integrity to the network.

Hydrogels have shown promise in biomedical and pharmaceutical applications, due, in part, to their high water content and rubbery or pliable nature, which may mimic natural tissue and may facilitate the release of bioactive substances at a desired physiological site. For example, hydrogels have been used or proposed for use in a variety of tissue treatment applications, including implants, tissue adhesives, bone grafts as well as in meniscus and articular cartilage replacement. Hydrogels may also act as a carrier for delivering bioactive substances including drugs, peptides, and proteins to a physiological site. Hydrogels have been made from a variety of hydrophilic polymers and copolymers. Poly(ethylene glycol), poly(vinyl pyrrolidone), polyacrylamide, poly(hydroxyethyl methacrylate), and copolymers of the foregoing, are examples of polymers that may be used to make hydrogels. Hydrogels have also been made from biopolymers such as chitosan, agarose, hyaluronic acid and gelatin, in addition from semi-interpenetrating network ("IPN") hydrogels and gelatin crosslinked with poly(ethylene glycol) diacrylate. Poly(vinyl alcohol) ("PVA") has been studied extensively for potential biomedical applications. PVA hydrogels can be produced, for example, from an aqueous solution via repeated freezing and thawing cycles that increase the order of the crystals, changing the dissolution properties, mesh size, and diffusion properties of the polymer. Peppas, et al., Adv. Polymer Sci. 153, 37 (2000), which is incorporated by reference in its entirety, provides an overview of developments in PVA hydrogels.

The present invention provides water-swellable materials including polymeric fibers dispersed in a water-swellable polymer, and fiber-reinforced articles made from such water-swellable materials. In embodiments of the present invention, the water-swellable material may have a polymer content, including the crosslinked polymeric fibers, of at least 30 wt %. Other embodiments have a polymer content of at least 25 wt %. In one embodiment, the crosslinked polymeric fibers constitute between about 1 wt % and about 10 wt % of the total polymer content.

The present invention also provides a method of forming a water-swellable material, which includes combining crosslinked polymeric fibers and a hydrophilic polymer with a carrier to form a water-swellable material in which the polymeric fibers are dispersed in the hydrophilic polymer, processing the water-swellable material to form a fiber-reinforced article and optionally crosslinking the fiber-reinforced article.

The water-swellable material of the present invention may be further processed to form fiber-reinforced articles or implants for use in a variety of biomedical and pharmaceutical applications and may be particularly suitable for implanted joint repair materials.

The invention will now be further described by way of example only with reference to the accompanying drawings in which:
Figure 1 is a digital image of a scanning electron micrograph of Example 2 showing residual fibers that are intact after processing.
Figure 2 is a digital image of a scanning electron micrograph of Example 9 showing residual fibers that are intact after processing.

The present invention generally provides water-swellable materials including polymeric fibers dispersed in a hydrophilic polymer, and fiber-reinforced articles made from such water-swellable materials. As used herein, the term "water-swellable" indicates a material able to absorb or adsorb and retain water upon hydration. In particular, some materials are able to absorb or adsorb and retain from about 5 % to about 95 % water and other materials absorb or adsorb and retain from about 20 % to about 80 % water.

The water-swellable material may be prepared by combining a hydrophilic polymer, crosslinked polymeric fibers and a suitable carrier. Suitable polymers for forming the water-swellable material include hydrophilic polymers and polymers derived from hydrophilic polymers including hydrogels. Suitable hydrophilic polymers include poly(vinyl alcohol), poly(glycols) such as poly(ethylene glycol) dimethacrylate, poly(ethylene glycol) diacrylate, poly(hydroxyethyl methacrylate), poly(vinyl pyrrolidone), poly(acrylamide), poly(acrylic acid), hydrolyzed poly(acrylonitrile), poly(ethyleneimine), ethoxylated poly(ethyleneimine) and poly(allylamine) as well as, monomers, oligomers, macromers, copolymers and/or other derivatives of the foregoing. The polymer materials reported in U.S. Application No. 11/358,383 entitled "Blend Hydrogels and Methods of Making," may also be suitable for use in certain embodiments. In alternative embodiments, hydrophilic biopolymers and IPNs may also be suitable. Other suitable polymers include polymers of poly(vinyl alcohol), poly(glycols), poly(ethylene glycol) dimethacrylate, poly(ethylene glycol) diacrylate, poly(hydroxyethyl methacrylate), poly(vinyl pyrrolidone), poly(acrylamide), poly(acrylic acid), hydrolyzed poly(acrylonitrile), poly(ethyleneimine), ethoxylated poly(ethyleneimine), poly(allyl alcohol), poly(allylamine), biopolymers such as chitosan, agarose, hyaluronic acid, collagen and gelatin, (semi) interpenetrating network hydrogels, peptide, protein, and blends and mixtures thereof.

Poly(vinyl) alcohol may be particularly suitable for use in certain embodiments. Poly(vinyl alcohol) for commercial use is generally produced by free-radical polymerization of vinyl acetate to form poly(vinyl acetate), followed by hydrolysis to yield PVA. The hydrolysis reaction does not go to completion, which leaves pendant acetate groups at some points along the polymer chain. In practice PVA can therefore be considered, in part, a copolymer of vinyl acetate and vinyl alcohol. The extent of the hydrolysis reaction determines the degree of hydrolysis of the PVA. Commercially available PVA can have a degree of hydrolysis over 98% in some cases.

In alternate embodiments of the present invention, the water-swellable precursor may be a polymer blend including a first polymer, which is a hydrophilic polymer such as poly(vinyl alcohol), and a second polymer having both hydrophobic and hydrophilic characteristics. For example, the second polymer may be a copolymer having hydrophobic recurring units and hydrophilic recurring units. By including the second polymer, it may be possible to vary or adjust the "stiffness" of the subsequently formed article.

In some embodiments, the hydrophobic recurring units include an aliphatic hydrocarbon segment. Aliphatic hydrocarbon recurring units may include -[CH₂CH₂-] or -[CH₂CH(CH₃)-], for example. In other embodiments, hydrophobic recurring units may include aliphatic, cyclic, or aromatic hydrocarbon pendant groups (e.g., pendant phenyl groups), or heterocyclic or heteroaromatic pendant groups. By way of example only, the hydrophobic region may also include fluorocarbon segments, segments including cyano pendant groups, or segments including imide groups.

In one embodiment, a majority of the hydrophobic recurring units have the structure - [CH₂CH₂-]. As used herein, the term "majority" means at least 50%. In another embodiment, the hydrophobic recurring units are predominantly of the form -[CH₂CH₂-]. As used herein, the term "predominantly" means a high proportion, generally at least 90%.

The hydrophilic recurring units of the polymer include recurring units having hydrophilic groups, such as hydroxyl pendant groups, carboxylic acid or sulfonic acid pendant groups, hydrophilic heterocyclic groups such as pyrrolidone pendant groups, or alkylene oxide groups (e.g., (C₁-C₆) alkylene oxide groups, more typically (C₁-C₃) alkylene oxide groups, such as -[CH₂O-], -[CH₂CH₂O-], -[CH(CH₃)O-], -[CH₂CH₂CH₂O-], -[CH(CH₃)CH₂O-], -[CH₂CH(CH₃)O-]) in the polymer backbone or as pendant groups.

In one embodiment, a majority of the hydrophilic recurring units include pendant -OH groups. In another embodiment, the hydrophilic recurring units predominantly include pendant -OH groups. In one embodiment, a majority of the hydrophilic recurring units have the structure -[CH₂CH(OH)-]. In another embodiment, the hydrophilic recurring units predominantly have the structure -[CH₂CH(OH)-].

In another embodiment, the second polymer is a copolymer derived from a hydrophobic monomer and a hydrophilic monomer having recurring units, for example, -[CH₂CH₂-] and recurring units of -[CH₂CH(OH)-]. In another embodiment, the copolymer includes recurring units of -[CH₂CH₂-] and recurring units of -[CH₂CH(OH)-] in a ratio in the range from about 1:1 to about 1:3.

One example of a suitable copolymer for use as the second polymer is poly(ethylene-co-vinyl alcohol), also known as "EVAL," "PEVAL" or "EVOH." Poly(ethylene-co-vinyl alcohol) may be formed into a hard, crystalline solid and is used commercially in food packaging and other applications. Commercially available grades of poly(ethylene-co-vinyl alcohol) are suitable for use in the present invention. Commercially available grades have an ethylene content, expressed as a mole-percent, of 26%, 27%, 28%, 29%, 32%, 35%, 44%, and 48%.

Examples of other copolymers having hydrophilic recurring units and hydrophobic recurring units include poly(ethylene-co-acrylic acid), poly(ethylene-co-methacrylic acid) and poly(styrene-co-allyl alcohol). Poly(styrene-co-allyl alcohol) having an average molecular weight of about 1600 may be particularly suitable. Diol-terminated poly(hexamethylene phthalate) may also be suitable for certain embodiments.

In a further embodiment, the second polymer may be a block copolymer having hydrophobic blocks and hydrophilic blocks. Suitable block copolymers may be derived from oligomers or prepolymers having hydrophobic and hydrophilic segments.

The polymer blend may generally include between about 5 wt % and about 95 wt % of the first polymer and between about 5 wt % and about 95 wt % of the second polymer. In some embodiments, the blend may include between about 30 wt % and about 95 wt % of the first polymer and between about 5 wt % and about 70 wt % of the second polymer. In other embodiments, the blend may include between about 50 wt % and about 95 wt % of the first polymer and between about 5 wt % and about 50 wt % by weight of the second polymer.

In a further embodiment, the hydrophilic polymer is a thermoplastic, which can be melted and re-solidified without losing its water-swellable character. In one embodiment, the material is a thermoplastic having a melting temperature in the range from about 70° C to about 200° C. The thermoplastic quality of the water-swellable material allows for easy processability and end use. Upon melting, the material becomes flowable and can therefore be extruded, injected, shaped, or molded.

The hydrophilic polymers reported above are combined with crosslinked polymeric fibers in the presence of a suitable carrier. Suitable polymeric fibers include non-woven, short chopped fibers, which are commercially available from a variety of sources. Examples of suitable synthetic fibers include polyvinyl alcohol (PVA), polyethylene terephthalate (PET), polyimide (PI) and polyetheretherketone (PEEK). Suitable natural fibers may be formed from collagen, chitin, chitosan, and the like. Suitable biodegradable fibers include poly(glycolic acid) (PGA), poly(lactic acid) (PLA), poly(lactic-co-glycolide) (PLG) copolymers, poly(glycolide-co-lactide) (PGL) copolymers, polydioxanone, and the like. Suitable inorganic fibers include, for example, carbon fibers, ceramic fibers, hydroxyapatite, polysiloxane fibers, and the like. Commercially available PVA fibers, Kuralon^{®} REC series (Kuraray Co. Ltd., Japan) are suitable for use in some embodiments and have an average diameter of 0.014 mm - 0.66 mm with an average length of 4 mm - 30 mm.

Prior to being combined with the hydrophilic polymer, the polymeric fibers may be crosslinked using irradiation or other conventional methods. In one embodiment, for example, the polymeric fibers may be gamma irradiated at 25 kGy prior to being combined with the hydrophilic polymer. In other embodiments, the polymeric fibers may be gamma irradiated at 50 kGy. Such crosslinking may improve the durability, preserve the crystallinity, and prevent dissolution of the fibers during subsequent processing steps, and in particular may prevent or reduce the breakdown of fibers in high temperature conditions.

In certain embodiments, the polymeric fibers are formed from the same polymer materials from which the hydrophilic polymer is derived. For example, both the hydrophilic polymer and the polymeric fibers may be formed or derived from poly(vinyl) alcohol.

The hydrophilic polymer and the polymeric fibers may be combined in the presence of a suitable carrier such that at least a portion of the hydrophilic polymer dissolves or plasticizes in the presence of the carrier. Suitable carriers include water, organic solvents or mixture thereof, as well as other plasticizers and diluents. Examples of suitable carriers include polar glycerine, ethylene glycol, propylene glycol, ethanol, tetrahydrofuran, toluene, dimethylformamide, dimethylacetamide, acetone, acetonitrile, cyclohexane, cyclopentane, 1,4-dioxane, ethyl acetate, glyme, methyl tert-butyl ether, methyl ethyl ketone, pyridine, and chlorobenzene.

One example of a suitable carrier is dimethyl sulfoxide (DMSO). In one embodiment, the carrier includes at least about 97 wt % DMSO. In another embodiment, the carrier includes about 70 to about 80 parts by weigh DMSO and about 20 to about 30 parts by weigh water. In some embodiments, the solvent includes essentially all DMSO and no water.

In some embodiments, a minor amount of water may be used in the carrier to aid in the formation of the water-swellable material to inhibit undesired phase separation and to achieve suitable crosslinking during a crosslinking step.

In some embodiments, the carrier may include saline or other physiological solution. The use of saline in manufacturing PVA hydrogels is described, for example, by Hassan, et al., J. Appl. Poly. Sci. 76, 2075 (2000). PVA hydrogels made in the presence of saline were reported to exhibit enhanced swelling kinetics and greater overall water content.

The hydrophilic polymer, polymeric fibers and carrier may be combined in several ways to form a water-swellable material. In one embodiment, the water-swellable material is formed by adding the hydrophilic polymer, polymeric fibers and any additives to the carrier to form a solution. A suitable amount, e.g. no greater than about 25 wt %, of the hydrophilic polymer is added to the carrier such that the polymer dissolves in the carrier. However, the polymeric fibers may not dissolve in the carrier, particularly if previously crosslinked, but may swell. Heating and continuously mixing the solution may assist in dissolution of the hydrophilic polymers and distribution of the fibers. The polymer-to-solvent ratio can vary widely using this method, but polymer dissolution is generally most effective if the hydrophilic polymer added is no more than 25 wt %.

In another embodiment, the water-swellable material is combined by compounding the appropriate hydrophilic polymer, polymeric fibers and a carrier in a heated mixing device such as a twin-screw compounder, sigma blade mixer, or twin-screw rheometer. Suitable temperatures for the compounding process depend on the materials to be combined. In one embodiment, the processing temperature ranges from about 80° C to about 130° C. In other embodiments, the processing temperature ranges from about 90° C to about 120° C. One benefit to compounding the mixture is a higher total polymer content, including the polymeric fibers, may be achieved as compared to solvent dissolution methods because the material acts as a plasticizer as opposed to a solution. In some embodiments, the total polymer content of the mixture may be at least about 25 wt %, in other embodiments it may be at least about 30 wt %.

After forming the water-swellable material, as reported above, further processing steps may be carried out to form fiber-reinforced (and water-swellable) articles. If the water-swellable material was formed as a solution, for example, conventional solution casting methods may be employed. Alternatively, if the water-swellable material was formed via the compounding process reported above, the material may be shaped into a desired article via conventional molding techniques, including compression, injection or liquid injection molding. Temperature and injection pressures depend on the materials and the mold design. In some embodiments, the temperatures may range from about 90° C to about 150° C. In other embodiments, the temperature is in the range from about 115° C to about 140° C. In another embodiment, the compression molding temperature range is from about 90° C to about 150° C with pressures of less than 4 tonnes.

In certain embodiments, the water-swellable material may be first formed into an intermediate form such as pellets, and then the pellets may later be melted and molded into a desired fiber-reinforced article. This method may be particularly suitable for use with water-swellable materials incorporating a thermoplastic polymer, which can be melted and re-solidified without losing its water-swellable character.

In some embodiments, after the water-swellable materials are further processed, for example by compounding or solution casting, the water-swellable materials are first immersed in alcohol followed by immersion in water. Depending on the type of processing used, the water-swellable materials may appear white and opaque or translucent. The fibers may be visible to the naked eye. The fibers are present as discrete fibers, that is, the fibers are separate and distinct and may be intact. In some embodiments, the fibers are randomly oriented, and in other embodiments, the fibers have some alignment.

The water-swellable material may be shaped into a variety of three-dimensional forms such as cylindrical derivatives or segments, spherical derivatives or segments, or polyhedral derivatives or segments.

In certain embodiments, the water-swellable material may be characterized by either low heat capacity or poor thermal conductivity, and may be manually handled in a heated, flowable state without special precautions. Melt-processability allows the water-swellable material to be manipulated so that *in situ* delivery and shaping may be accomplished. Therefore, the water-swellable material may be directly injected into the body of a patient, for example, by employing a hot gun, to allow for *in situ* shaping of a water-swellable material that may then be attached or bonded at the site. Such a technique may have practical applications in minimally invasive surgical procedures.

Optionally, the water-swellable materials or fiber-reinforced articles of the present invention may be subjected to one or more crosslinking steps. Crosslinking may be carried out after forming the water-swellable material, after shaping the material into a fiber-reinforced article, after *in situ* formation of the fiber-reinforced article, or at any other suitable point during processing.

A variety of conventional approaches may be used to crosslink the water-swellable material, including, physical crosslinking (e.g., freeze thaw method), photoinitiation, irradiation and chemical crosslinking.

Crosslinking by electron-beam or gamma irradiation, such as by using a Co⁶⁰ source, may be employed according to embodiments of the present invention.

Physical crosslinking may be achieved by conventional techniques described in, for example, Peppas, et al., Adv. Polymer Sci. 153, 37 (2000), incorporated herein by reference in its entirety.

In one embodiment, the water-swellable material may be subjected to at least one freeze-thaw cycle, which may result in at least partial physical crosslinking of the polymer blend to produce a crosslinked blend.

With respect to PVA polymers, repeated cycles of freezing at -20° C and thawing at 25° C result in the formation of crystalline regions that remain intact upon being placed in contact with water or biological fluids at 37° C. Similar or identical parameters for freeze-thaw cycles are suitable in the practice of the present invention.

In other embodiments of the invention, the water-swellable material may be chemically crosslinked. Examples of suitable chemical crosslinking agents include monoaldehydes such as formaldehyde, acetaldehyde, or glutaraldehyde in the presence of a solvent such as methanol. Other suitable crosslinking agents include diisocyanate compounds, which can result in urethane linkages, or epoxy compounds. Crosslinking achieved using enzymes such as a calcium independent microbial transglutaminase, which catalyzes transamidation reactions to form N-∈-(γ-glutamyl)lysine crosslinks in proteins, may also be suitable according to embodiments of the present invention.

A combination of crosslinking techniques may also be utilized in the invention. For example, a freeze-thaw cycle could be used to provide physical crosslinking, followed by irradiation or photoinitiation to provide more complete crosslinking. As other examples, chemical crosslinking could be followed by irradiation, or photoinitiation or a freeze-thaw step could be performed after crosslinking by any of chemical, irradiation or photoinitiation.

In one embodiment, irradiation may be used to selectively crosslink regions of the water-swellable or fiber-reinforced article material in a variety of geometries, patterns or gradients. This selective crosslinking may be used to customize and tailor the mechanical and physical characteristics of the fiber-reinforced article. This crosslinking process may be used either during production of the fiber-reinforced article or may be used *in situ* after the article is placed in contact with a soft tissue at a desired site or location, such as at a joint repair site of a hip, knee, spine, finger, elbow, ankle, toe or shoulder. In certain applications in which sterilization is desired, crosslinking may be combined with sterilization into a single step.

The water-swellable materials of the present invention can be used in a variety of applications, including minimally invasive surgical procedures. For example, the water-swellable materials may be used to prepare artificial articular cartilage, as artificial meniscus or articular bearing components, and in the repair of the temporomandibular joint, proximal interphalangeal joint, metacarpophalangeal joint, metatarsalphalanx joint, or hip capsule joint.

The water-swellable materials of the present invention could also be used to replace or rehabilitate the nucleus pulposus of an intervertebral disc in certain embodiments. Degenerative disc disease in the lumbar spine is marked by a dehydration of the intervertebral disc and loss of biomechanical function of the spinal unit. A recent approach has been to replace only the central portion of the disc, called the nucleus pulposus.

The water-swellable material or fiber-reinforced article can also be employed in a spinal disc prosthesis used to replace a part or all of a natural human spinal disc. By way of example, a spinal disc prosthesis may include a flexible nucleus, a flexible braided fiber annulus, and end-plates. The water-swellable material or fiber-reinforced article may be employed in the flexible nucleus, for example.

The ability of water-swellable materials to release therapeutic drugs or other active agents has been reported. The water-swellable material of the present invention may also be employed *in vivo* to provide elution of a protein, drug, or other pharmacological agent impregnated in the water-swellable material or provided on the surface of a fiber-reinforced articles. The additional optional additives for the water-swellable material include growth factors, analgesics, antibiotics, stem cells, or osteochondral cells.

### EXAMPLES

### General Procedures and Processes for Examples 1-23

The general procedures and processes for making fiber-reinforced hydrogels (Examples 1-23) are described below. Table 1 shows the amount of each material used in the Examples. The amount of PVA and PVA fibers are provided in grams, the water and DMSO in milliliters and the fiber diameter in denier and length in millimeters. The fibers were irradiated using gamma irradiation at Sterigenics (Charlotte, NC) at either 25 +/- 3 kGy or 50+/- 3 kGy dose.

**Table 1. Fiber-Reinforced Hydrogel Examples 1-23**

| **Fiber-Reinforced Hydrogel Example** | **PVA** | **Poly(ethylene-co-vinyl alcohol)** | **Water** | **DMSO** | **Fiber** | **Fiber (Diameter X Length)** | **Dose** | **% PVA** | **% Fiber** | **Post Crosslinked** |
|---|---|---|---|---|---|---|---|---|---|---|
| 1 | 33.25 | - | 7 | 28 | 1.75 | 15 X18 | 25 | 47.50 | 2.50 | - |
| 2 | 33.25 | - | 7 | 28 | 1.75 | 100 X 12 | 25 | 47.50 | 2.50 | - |
| 3 | 34.65 | - | 7 | 28 | 0.35 | 15 X18 | 50 | 49.50 | 0.50 | - |
| 4 | 33.25 | - | 7 | 28 | 1.75 | 100 X 12 | 50 | 47.50 | 2.50 | - |
| 5 | 20 | - | 9.8 | 39.2 | 1 | 100 X 12 | 25 | 28.57 | 1.43 | - |
| 6 | 33.25 | - | 7 | 28 | 1.75 | 7 X 6 | 50 | 47.50 | 2.50 | - |
| 7 | 31.5 | - | 7 | 28 | 3.5 | 15 X18 | 50 | 45.00 | 5.00 | - |
| 8 | 33.25 | - | 7 | 28 | 1.75 | 15 X18 | 50 | 47.50 | 2.50 | - |
| 9 | 16 | - | 8.4 | 33.6 | 12 | 15 X18 | 25 | 22.86 | 17.14 | - |
| Comparative 10 | 35 | - | 7 | 28 | 0 | - | - | 50.00 | - | - |
| 11 | 35 | - | 7 | 28 | 0 | - | - | 50.00 | - | - |
| 12 | 31.5 | - | 6.3 | 25.2 | 10 | 100 X 12 | 50 | 43.15 | 13.70 | - |
| 13 | 14 | 17.5 | 7 | 28 | 3.5 | 7 X 6 | 50 | 20.00 | 5.00 | - |
| Comparative 14 | 14 | 17.5 | 7 | 28 | 0 | - | 50 | 21.05 | - | - |
| 15 | 33.25 | - | 7 | 28 | 1.75 | 100 X 12 | 25 | 47.5 | 2.5 | Freeze Thaw |
| 16 | 34.65 | - | 7 | 28 | 0.35 | 15 X 18 | 50 | 49.5 | 0.5 | Freeze Thaw |
| 17 | 33.25 | - | 7 | 28 | 1.75 | 15 X 18 | 25 | 47.5 | 2.5 | Irradiation |
| 18 | 33.25 | - | 7 | 28 | 1.75 | 100 X 12 | 25 | 47.5 | 2.5 | Irradiation |
| 19 | 34.65 | - | 7 | 28 | 0.35 | 15 X 18 | 50 | 49.5 | 0.5 | Irradiation |
| 20 | 33.25 | - | 7 | 28 | 1.75 | 100 X 12 | 50 | 47.5 | 2.5 | Irradiation |
| 21 | 33.25 | - | 7 | 28 | 1.75 | 7 X 6 | 50 | 47.5 | 2.5 | Irradiation |
| 22 | 31.5 | - | 7 | 28 | 3.5 | 15 X 18 | 50 | 45 | 5.0 | Irradiation |
| 23 | 33.25 | - | 7 | 28 | 1.75 | 15 X 18 | 50 | 47.5 | 2.5 | Irradiation |

To a Haake Polylab^{®} twin screw rheometer was added PVA, water, DMSO, and PVA fiber. The materials were mixed at 120°C for 5 minutes. The PVA, obtained from Sigma Aldrich, is 99+% hydrolyzed with an average molecular weight of 146,000 to 186,000kDa. The poly(ethylene-covinyl alcohol) was used as received from Sigma Aldrich and contained 44% ethylene. The PVA fibers used are the Kuralon^{®} REC series available from Kuraray Co. Ltd. (Japan). The PVA fibers were irradiated prior to use. The DMSO, obtained from Sigma Aldrich, contained ≤0.4% water.

After mixing for 5 minutes, the sample was removed, cooled to room temperature, and chopped into flake form for use in the Battenfeld BA 100 CD injection molder machine. The resulting material remained translucent, flexible, and pliable.

### Examples 1-14

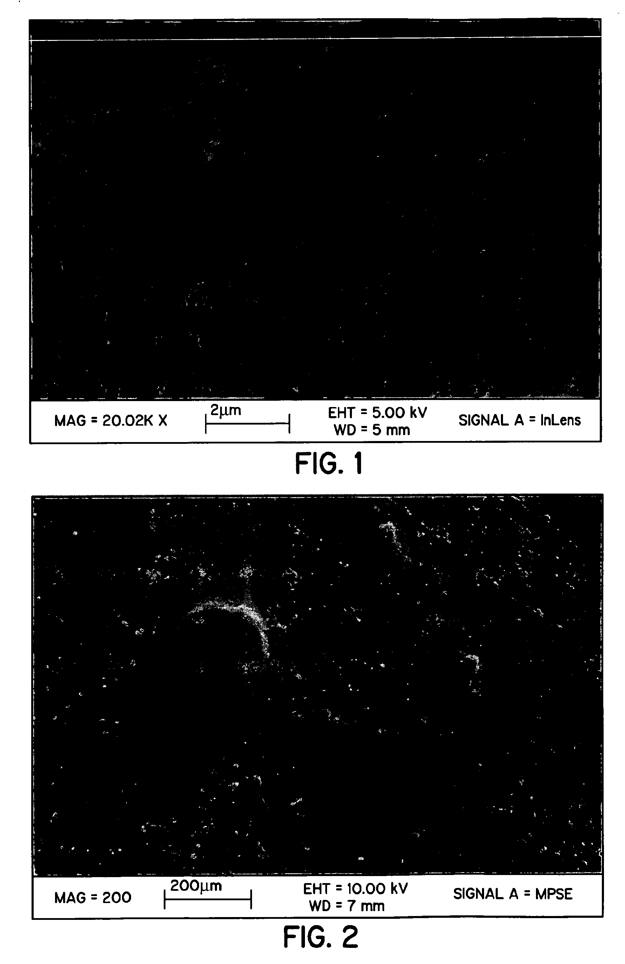

The translucent, flexible, and pliable material obtained from Examples 1-10 were further processed on a Battenfeld BA 100 CD injection molder with nozzle temperatures between 240°F - 280°F (115° C - 138° C) and the mold at room temperature. Samples from injection molding were first immersed in alcohol for a minimum of 20 minutes followed by immersion in water. Samples 1-10 were immersed in 80°C water for 20 minutes followed by room temperature water for 2 days. FIG. 1 and FIG. 2 show the incorporation of fibers into the gel matrix for Samples 2 and 9, respectively. Samples 11-14 were immersed only in room temperature water for 2 days. Fibers could be seen by the naked eye. Some fiber alignment was present in the direction of melt flow.

### Example 15

The water-swellable material obtained from Example 2 was processed on a Battenfeld BA 100 CD injection molder to form a tensile bar and compression molded sample specimens. The specimens were immersed in alcohol for a minimum of 20 minutes followed by immersion in 80°C water for 20 minutes. The samples were then allowed to solvent exchange in deionized water at room temperature for 2 days. The samples were exposed three times to a repetitive freeze-thaw cycle. In the cycle, the samples were frozen by placing in a freezer at -30°C for 12 hours followed by thawing at room temperature for 12 hours.

### Example 16

The water-swellable material obtained from Example 3 was processed as described in Example 15.

### Example 17

The water-swellable material obtained from Example 1 was nitrogen-packed and irradiated at 75 kGy at Sterigenics in Charlotte, North Carolina. Samples were then allowed to rehydrate for one day in deionized water prior to testing.

### Example 18

The water-swellable material was obtained from Example 2 was processed as described in Example 17.

### Example 19

The water-swellable material obtained from Example 3 was processed as described in Example 17.

### Example 20

The water-swellable material obtained from Example 4 was processed as described in Example 17.

### Example 21

The water-swellable material obtained from Example 6 was processed as described in Example 17.

### Example 22

The water-swellable material obtained from Example 7 was processed as described in Example 17.

### Example 23

The water-swellable material obtained from Example 8 was processed as described in Example 17.

Mechanical performance properties for selected hydrogels were measured using the American Society of Testing Materials standards (ASTM D638 Type IV specimens) and using conventional techniques on a Model 3345 from Instron Corporation. Tensile specimens were kept hydrated during the test using a peristaltic pump with a rate of 60 drops per second. Compression testing was performed on a Model 3345 from Instron Corporation in a water bath at room temperature. Compression samples were in cylinders of 0.25 x 0.25 inches. Measured values for tensile properties are reported in Table 2. Measured values for compressive properties are reported in Table 3. Tensile properties for examples 17-23 are reported in Table 4.

**TABLE 2. Tensile properties for selected crosslinked fiber hydrogels.**

| | **Stress at Peak (psi)** | **Percent Strain at Peak** | **Stress at Break (psi)** | **Percent Strain at Break** | **Young's Modulus (ksi)** | **Energy at Yield (Ibf-in)** | **Energy at Break (Ibf-in)** |
|---|---|---|---|---|---|---|---|
| Example 1 | 366 | 199 | 259 | 224 | 0.29 | 0.52 | 2.70 |
| Example 2 | 500 | 168 | 296 | 190 | 0.43 | 0.97 | 2.92 |
| Example 3 | 418 | 169 | 301 | 186 | 0.34 | 0.39 | 2.41 |
| Example 4 | 434 | 133 | 281 | 157 | 0.42 | 0.64 | 2.23 |
| Example 5 | 186 | 340 | 131 | 370 | 0.11 | 0.90 | 2.14 |
| Example 6 | 462 | 145 | 274 | 166 | 0.39 | 0.74 | 2.54 |
| Example 7 | 356 | 136 | 276 | 154 | 0.60 | 0.96 | 1.91 |
| Example 8 | 466 | 158 | 283 | 183 | 0.37 | 0.75 | 2.79 |
| Example 9 | 315 | 312 | 167 | 347 | 0.16 | 1.69 | 2.86 |
| Example 10 (Control for 1-4,6-8) | 191 | 277 | 107 | 317 | 0.17 | 1.63 | 2.27 |
| Example 11 | 450 | 278 | 338 | 278 | 0.29 | 1.39 | 3.63 |
| Example 12 | 694 | 176 | 437 | 208 | 0.55 | 1.07 | 3.37 |
| Example 13 | 1074 | 305 | 871 | 322 | 664.40 | 2.46 | 7.76 |
| Example 14 (Control for 12) | 831 | 555 | 717 | 57 | 344.92 | 9.19 | 9.59 |

**TABLE 3. Selected compressive properties of crosslinked fiber hydrogels.**

| | **Compressive Tangent Modulus at Different Strain Levels (psi)** | | | | |
|---|---|---|---|---|---|
| | **20% Strain** | **30% Strain** | **40 % Strain** | **60% Strain** | **70 % Strain** |
| Example 1 | 823.9 | 976.4 | 1286.9 | 3018.3 | 8035.6 |
| Example 2 | 751.8 | 912.4 | 1225.7 | 3303.3 | 8416.9 |
| Example 3 | 649.4 | 832.9 | 1150.7 | 2649.6 | 6630.4 |
| Example 4 | 868.4 | 1030.4 | 1382.1 | 3535.0 | 8841.5 |
| Example 5 | 531.2 | 656.6 | 872.2 | 1958.2 | 5167.2 |
| Example 6 | 1060.2 | 1227.3 | 1653.0 | 4584.6 | 10431.2 |
| Example 7 | 804.3 | 913.9 | 1242.6 | 3045.2 | 7426.5 |
| Example 8 | 898.4 | 1026.2 | 1349.8 | 3459.2 | 8260.7 |
| Example 9 | 549.6 | 690.9 | 787.9 | 1839.8 | 4581.6 |
| Example 10 (Control for 1-4,6-8) | 331.5 | 478.0 | 730.3 | 2036.9 | 5029.4 |
| Example 11 | 2302.1 | 2180.3 | 2230.3 | 3136.4 | -- |
| Example 12 (Control for 11) | 1670.2 | 1770.0 | 1860.8 | 2806.5 | 4737.0 |
| Example 13 | 2302.1 | 2180.3 | 2230.3 | 3136.4 | -- |
| Example 14 (Control for 13) | 1670.2 | 1770.0 | 1860.8 | 2806.5 | 4737.0 |
| Example 15 | 651.6 | 862.6 | 1242.4 | 3082.6 | 7200.0 |
| Example 16 | 527.4 | 747.3 | 1094.4 | 2516.9 | 1215.4 |

**TABLE 4. Tensile properties for selected crosslinked fiber hydrogels after 75 kGy post irradiation.**

| | **Stress at Peak (psi)** | **Percent Strain at Peak** | **Stress at Break (psi)** | **Percent Strain at Break** | **Young's Modulus (ksi)** | **Energy at Yield (lbf-in)** | **Energy at Break (lbf-in)** |
|---|---|---|---|---|---|---|---|
| Example 17 | 277 | 120 | 113 | 156 | 0.32 | 0.21 | 1.13 |
| Example 18 | 417 | 115 | 279 | 139 | 0.48 | 0.42 | 1.50 |
| Example 19 | 456 | 148 | 324 | 163 | 0.58 | 0.64 | 2.29 |
| Example 20 | 494 | 123 | 324 | 143 | 0.52 | 0.53 | 2.05 |
| Example 21 | 526 | 146 | 322 | 163 | 0.46 | 0.73 | 2.33 |
| Example 22 | 210 | 85 | 92 | 122 | 0.35 | 0.09 | 0.81 |
| Example 23 | 315 | 83 | 208 | 107 | 0.53 | 0.30 | 1.11 |

The results set forth in Tables 2, 3, and 4 indicate that samples containing crosslinked fibers possessed certain improved mechanical characteristics over the control samples. The data showed that the material had become stiffer, showed less elongation and was more crosslinked after post irradiation.

This invention may take on various modifications and alterations. In describing embodiments of the invention, specific terminology is used for the sake of clarity. The invention, however, is not intended to be limited to the specific terms so selected, and it is to be understood that each term so selected includes all technical equivalents that operate similarly.

## Claims

1. An implantable article comprising a fiber-reinforced, water-swellable material including a plurality of crosslinked polymeric fibers dispersed within a water-swellable polymer.

2. The article of claim 1, wherein the water-swellable polymer comprises a hydrophilic polymer.

3. The article of claim 1, wherein the water-swellable polymer comprises a homopolymer.

4. The article of claim 1, wherein the water-swellable polymer comprises a hydrophilic polymer and a hydrophobic polymer.

5. The article of claim 1, wherein the water-swellable polymer comprises a blend of polymers.

6. The article of claim 1, wherein the water-swellable polymer comprises a thermoplastic material.

7. The article of claim 1, wherein the water-swellable polymer comprises a copolymer.

8. The article of claim 1, wherein the water-swellable polymer is selected from the group consisting of biopolymers, chitosan, agarose, hyaluronic acid, collagen, gelatin, (semi) interpenetrating network hydrogels, peptide, and protein, and blends and combinations thereof.

9. A fiber-reinforced, water-swellable article comprising a water-swellable hydrophilic polymer, and a plurality of crosslinked polymeric fibers dispersed within the water-swellable, hydrophilic polymer.

10. The article of either claim 1 or claim 9, wherein the water-swellable hydrophilic polymer is selected from the group consisting of polymers of poly(vinyl alcohol), poly(glycols), poly(ethylene glycol) dimethacrylate, poly(ethylene glycol) diacrylate, poly(hydroxyethyl methacrylate), poly(vinyl pyrrolidone), poly(acrylamide), poly(acrylic acid), hydrolyzed poly(acrylonitrile), poly(ethyleneimine), ethoxylated poly(ethyleneimine), poly(allyl alcohol) and poly(allylamine), and combinations thereof.

11. The article of either claim 1 or claim 9, wherein the plurality of crosslinked polymeric fibers and the water-swellable hydrophilic polymer comprise the same polymer.

12. The article either claim 1 or claim 9, wherein the plurality of crosslinked polymeric fibers are gamma-irradiated, polyvinyl (alcohol) fibers.

13. The article of any preceding claim, wherein the plurality of crosslinked polymeric fibers are randomly oriented in the water-swellable polymer material.

14. The article of any preceding claim, wherein the article comprises a total polymer content of at least 25 weight percent.

15. The article of any preceding claim, wherein the article comprises a total polymer content of at least 30 weight percent.

16. The article of any preceding claim, wherein the crosslinked polymeric fiber comprises between about 1 weight percent and about 10 weight percent of the total polymer content of the article.

17. The article of any preceding claim, wherein the article comprises a joint repair material, an articulating or bearing surface in a hip, knee, spine, finger, elbow, toe, ankle or shoulder joint, or a spinal prosthesis.

18. A method of forming a fiber-reinforced article comprising:
combining crosslinked polymeric fibers and a hydrophilic polymer with a carrier to form a water-swellable material with the polymeric fibers dispersed in the hydrophilic polymer;
processing the water-swellable material to form a fiber-reinforced article.

19. The method of claim 18, wherein the processing comprises crosslinking the water-swellable material.

20. The method of either claim 18 or claim 19, wherein the hydrophilic polymer, the crosslinked polymeric fibers, and the carrier are combined at a temperature in the range of about 80 °C to about 130 °C.

21. The method of any one of claims 18 to 20 wherein the carrier comprises dimethyl sulfoxide.

22. The method of any one of claims 18 to 21 wherein the fiber-reinforced article comprises a total polymer content of at least 30 weight percent.

23. The method of any one of claims 18 to 22 wherein the fiber-reinforced article is processed by solution casting, compression molding, injection molding, or liquid injection molding.
